(19) **Europäisches Patentamt European Patent Office Office européen des brevets**

(11) **EP 2 144 637 B1**

(12) **EUROPEAN PATENT SPECIFICATION**

(45) Date of publication and mention
of the grant of the patent:
**29.06.2011 Bulletin 2011/26**

(51) Int Cl.:
***A61L 15/16*** (2006.01)     ***A61L 15/50*** (2006.01)

(21) Application number: **08751215.8**

(22) Date of filing: **15.05.2008**

(86) International application number:
**PCT/IB2008/051923**

(87) International publication number:
**WO 2008/139427 (20.11.2008 Gazette 2008/47)**

(54) **ABSORBENT ARTICLE WITH HYDROPHILIC LOTION AND HIGH-BARRIER CUFFS**

SAUGFÄHIGER ARTIKEL MIT HYDROPHILER LOTION UND HOCHGRADIGEN BARRIEREBÜNDCHEN

ARTICLE ABSORBANT POURVU D'UNE LOTION HYDROPHILE ET D'ÉLÉMENTS DE HAUTE PROTECTION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.05.2007 EP 07108199**
           **15.05.2007 EP 07108202**
           **28.11.2007 US 946631**

(43) Date of publication of application:
**20.01.2010 Bulletin 2010/03**

(73) Proprietor: **The Procter & Gamble Company**
**Cincinnati, OH 45202 (US)**

(72) Inventors:
• **VEGA, Victor, Nicholas**
  **Cincinnati, Ohio 45231 (US)**
• **WISE, Brandon, Ellis**
  **Cincinnati, Ohio 45208 (US)**
• **ROSATI, Rodrigo**
  **60486 Frankfurt Am Main (DE)**

(74) Representative: **Heide, Ute et al**
**Procter & Gamble European Service GmbH**
**Sulzbacher Strasse 40-50**
**65824 Schwalbach am Taunus (DE)**

(56) References cited:
**WO-A-00/57843**      **WO-A-02/49686**
**WO-A-97/05909**      **WO-A-03/028776**
**WO-A-2007/044569**   **JP-A- 2006 297 071**
**US-A- 5 968 025**     **US-A1- 2002 016 579**
**US-A1- 2003 154 904**   **US-A1- 2006 025 737**
**US-A1- 2006 140 924**

**Description**

FIELD OF THE INVENTION

This invention is directed to an absorbent article to be worn by a wearer comprising a topsheet, a backsheet, an absorbent core and a hydrophilic lotion to reduce the adherence of the menses or feces to the skin, thereby improving the ease of menses or bowl movement (BM) clean up; the absorbent article further comprises one or more barrier cuffs to provide a high barrier to reduce leakage of liquids, such as urine. Preferred absorbent articles are infant (baby) diapers, including training pants, adult incontinence articles and the like.

BACKGROUND OF THE INVENTION

Disposable absorbent products, such as diapers and sanitary napkins, are known that have a topsheet comprising a lotion, to deliver skin benefits to the skin of the wearer and to sometimes improve the removal of feces or menses from the skin. In recent years the focus has been to deliver lotions to sanitary napkins and diapers that provide extra skin benefits, for example by addition of botanical ingredients or pharmaceutical ingredients to the lotions. Lotions of various types are known to provide various skin benefits, such as prevention or treatment of diaper rash. These lotions can be applied to the topsheet of absorbent articles, and can be transferred to the skin of the wearer during use. The addition of lotion to the topsheet of absorbent articles is also known to provide benefits such as easier BM clean up on the skin.

US 5,968,025 to Roe et al., WO 97/05908, WO 97/05909 and US 2006/140924 describe absorbent articles having lotioned topsheets for reducing adherence of BM to the skin, wherein the lotion compositions are primarily hydrophobic. US 3,489,148 to Duncan et al. teaches a diaper comprising a hydrophobic and oleophobic topsheet wherein a portion of the topsheet is coated with a discontinuous film of oleaginous material. However, in diapers disclosed in the Duncan et al. reference and other diapers treated with hydrophobic lotions, the hydrophobic and oleophobic topsheets are relatively slow in promoting transfer of urine to the underlying absorbent cores. Since the viscosity of BM and menses is higher then the viscosity of urine, the problems of slow transfer to the absorbent core is more profound. Accordingly, there is a continuing need for absorbent articles, such as diapers and catamenial devices having improved fluid handling such that more menses enter into and remain in the device, and less on the skin and hair of the wearer. WO 05/035013, WO 00/64500, WO 00/64501, U.S. Patent Application 2002-120241 and U.S. Patent 6,756,520 describe absorbent articles with hydrophilic lotion compositions for various uses, such as improving moisturization or lubrication, for reducing abrasion of skin, for improving skin health, for enhancing the barrier function of the skin and for prevention and alleviation of skin irritations.

Despite all these benefits often an increase in leakage is observed when hydrophilic lotions are used in absorbent articles, such as diapers. Without being bound by theory, one conceivable reason for the increase in leakage may be the reduction of the surface tension of the fluid, which is urine in the case of diapers, wetting the absorbent article after contact with the lotion. Furthermore it is believed that during diaper wear, hydrophilic lotion may transfer for example from the topsheet to the skin of the wearer and then from skin to the cuffs. Another reason for the transfer of ingredients of the lotion to the cuffs may be that it is washed of from for example the topsheet by liquids such as urine. Lotion contamination of these cuffs may then result in a lower fluid barrier, due to either surface tension reduction of urine or increased wetting of the cuff due to the presence of hydrophilic lotion. In particular when surface active substances are present in such hydrophilic lotions, the reduction of the surface tension of the bodily fluid, e.g. urine, may be observed. Since both features, easy BM clean up on the one hand and as little leakage as possible on the other hand, are important to the convenience of an absorbent article the need for providing an absorbent article possessing these both benefits arises.

It is the aim of the present invention to provide an absorbent article having the benefits of the use of hydrophilic lotions, such as reducing the adherence of the menses or feces to the skin and thereby improving the ease of menses or bowel movement (BM) clean up, and reducing the tendency of leakage of liquid, such as urine.

It has now been found by the inventors that by using the herein described high barrier cuffs in combination with the herein described hydrophilic lotion in an absorbent article the lotion can be applied in an effective amount and leakage is reduced.

SUMMARY OF THE INVENTION

The present invention relates to an absorbent article as mentioned in the claims.

DETAILED DESCRIPTION OF THE INVENTION

As used herein, the following terms have the following meanings:

Herein, "comprise" and "include" mean that other elements and/or other steps which do not affect the end result can be added. Each of these terms encompasses the terms "consisting of" and "consisting essentially of".

Herein, "body facing surface" refers to surfaces of absorbent articles and/or their component materials which face the body of the wearer, while "garment facing surface" refers to the opposite surfaces of the absorbent articles and/or their component materials that face away from the wearer when the absorbent articles are worn. Absorbent articles and components thereof, including the topsheet, backsheet, absorbent core, and any individual layers of their component materials, typically have a body facing surface and a garment facing surface.

Herein, "body" refers to outer layers formed by mammalian epidermal tissues including the skin and hair. The characteristics of the body tend to differ dramatically depending on the position, age, sex, and individual's nature. For example, the skin of babies and young children differs from the skin of adults, and the skin having hair differs from the non-haired skin.

As used herein "absorbent article" refers to devices which are intended to be placed against the skin of a wearer to absorb and contain the various exudates discharged from the body. Absorbent articles of the present invention include diapers, pant-like diapers and incontinence articles, and feminine hygiene articles.

As used herein "diaper" refers to an absorbent article generally worn by infants (e.g. babies or toddlers) about the lower torso of the wearer. Suitable diapers are disclosed in, e.g., U.S. Patent 3,860,003 issued to Buell on January 14, 1975; U.S. Patent 5,151,092 issued to Buell et al. on September 29, 1992; U.S. Patent 5,221,274 issued to Buell et al. on June 22, 1993; and U.S. Patent 5,554,145 issued to Roe et al. on September 10, 1996. As used herein the term "diaper" also comprises "pant-like diapers": A pant-like diaper refers to an absorbent article having fixed sides and leg openings. Pant-like diapers are placed in position on the wearer by inserting the wearer's legs into the leg openings and sliding the pant-like diaper into position about the wearer's lower torso. Suitable pant-like diapers are disclosed in, e.g., U.S. Patent No. 5,246,433, issued to Hasse, et al. on September 21, 1993.

As used herein "incontinence article" refers to an absorbent article worn about the lower torso of the wearer, pads, undergarments, inserts for absorbent articles, capacity boosters for absorbent articles, briefs, bed pads, and the like worn by incontinent persons that are typically adults. Suitable incontinence articles are disclosed in, e.g., U.S. Patent No. 4,253,461 issued to Strickland, et al. on March 3, 1981; U.S. Patent Nos. 4,597,760 and 4,597,761 issued to Buell; the above-mentioned U.S. Patent No. 4,704,115; U.S. Patent No. 4,909,802 issued to Ahr, et al.; U.S. Patent No. 4,964,860 issued to Gipson, et al. on October 23, 1990; and PCT Publication No. WO 92/11830 published by Noel, et al. on July 23, 1992.

As used herein "disposable" is used to describe absorbent articles for single use, which are not intended to be laundered, restored or otherwise reused as an absorbent article after a single use.

The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.

The term "longitudinal", as used herein, refers to a line, axis or direction in the plane of the absorbent article that is generally aligned with (e.g., approximately parallel to) a vertical plane which bisects a standing wearer into left and right body halves when the absorbent article is worn. The terms "transverse" or "lateral" as used herein, are interchangeable, and refer to a line, axis or direction which lies within the plane of the absorbent article that is generally perpendicular to the longitudinal direction.

As used herein, the terms "migrate", "migration", or "migrating" mean a lotion composition moves from one place to another place by way of movement on a material or permeation through an intervening material.

As used herein, the term "transfer" when used in the context of a lotion composition, refers to the lotion composition moving from one area of the absorbent article to the skin of the wearer or to another area on the absorbent article not by way of migration but by way of direct contact with the lotion composition, such as in a blotting effect.

"Applying" to a surface of an absorbent article such as the topsheet as used herein means that said surface, e.g. topsheet, comprises at least a partial layer of the lotion composition on at least part of one of its surface so that at least part of the lotion composition may contact the skin of the wearer in use. To allow contact with the skin, typically the body facing surface of the topsheet is coated. The lotion composition may also penetrate partly or fully into the surface and may penetrate the respective absorbent article component to which it is applied, e.g. the topsheet.

The unit of all molecular weights given herein is Daltons.

As used herein, the term "particulate material" refers to a component of the lotion composition that is insoluble or non-molecularly dispersible or non-reactive biologically in the lotion composition prior to applying this lotion composition to the absorbent article and that remains in particulate form when applied to the absorbent article. It includes all type of particulate forms such as granules, beads, spheres, micro-spheres, powders, as known in the art.

The terms "reducing the adherence" and "anti-stick" are used synonymously. This means that less residual bowel movement remains on the skin when compared to an article without anti-stick lotion composition.

Herein, the terms "feces" and "bowl movement", "BM" are used interchangeably.

Lotion compositions of the present invention

The lotion composition of the present invention is a hydrophilic lotion, having a water solubility of at least 40% (determined according to the method as described herein).

The lotion composition preferably is flowable (e.g. liquid) at suitable process conditions, e.g. above 50°C or above 60°C or above 80°C or optionally above 100°C, but solid or semi-solid at a temperature of 25 °C.

The lotion composition herein is preferably a lotion composition that may provide additional skin care benefits. The lotion compositions of the present invention are typically non-fluid, i.e. solid, or more often semisolid, at 25°C, i.e. at ambient temperatures to minimize migration of the lotion composition. By "semisolid" is meant that the lotion composition has a rheology typical of pseudoplastic or plastic fluids. When no shear is applied, the lotion compositions can have the appearance of a solid but can be made to flow as the shear rate is increased. This may be due to the fact that, while the lotion composition contains a component being solid at 25°C, it also includes a component being liquid at 25°C. Each component may itself comprise one or more compounds. The lotion composition preferably has a final melting point (more than 95% liquid) above potential "stressful" storage conditions that can be 45°C or greater. Semi-solid as used herein means that 1 g of the material, which is placed in the middle of a round glass plate having a diameter of 15 cm, does not run off a glass plate within 1 minute, when the glass plate is tilted at 45°, under conditions of 25°C and 50 % relative humidity. All components or compounds being either solid or at least semi-solid (according to the test-method described above) are herein referred to as "solid compound or solid component". All components or compounds being not solid or at least semi-solid (according to the test-method described above) are herein referred to as "liquid compound or liquid component".

In one embodiment, the lotion composition is such that 3% to 75% by weight is liquid at test temperature of 20 °C. In one embodiment the lotion according to present invention is such that 10% to 80% by weight, or 20% to 70% by weight, or 30% to 60% by weight, or 40% to 50% by weight is liquid at a test temperature of 25°C. From 25% to 75% or even 30% to 80% can be liquid at body temperature of 37 °C. In one embodiment, at 25°C the total amount of liquid compounds is higher than the total amount of solid compounds, e.g. the amount of liquids is above 50 wt.% or at least 55 wt.% or at least 60 wt.%. When applied to the absorbent article, the lotion compositions of the present invention are transferable to the wearer's skin by normal contact, wearer motion (thus creating friction), and/or body heat.

An amount of the lotion composition is comprised on a body facing surface of an absorbent article. The amount should effect a reduction of the adherence of feces or menses to the human skin of a wearer wearing an absorbent article compared to the absorbent article without the lotion composition. Without being bound by theory, it is believed that the lotion composition may reduce the adhesive force between the soils or exudates and the skin surface because the adhesive forces may be smaller than the cohesive forces within the soils or exudates, thereby allowing the soils or exudates to detach from the skin surface upon application of a shear force (e.g. such as that generated by wiping).

Typically, the lotion composition is applied on at least a portion of the topsheet. A suitable amount according to the present invention may be from about 0.015 grams per square meter (gsm) to about 100 gsm, or 1 gsm to about 80 gsm, or 6 gsm to 50 gsm, or 12 gsm to 40 gsm, or 16 to 30 gsm. For example 24 gsm.

Said first liquid component may comprise a liquid polyethylene glycol and said second component may comprise a solid nonionic surfactant with an HLB value of at least 10; or

said first component may comprise a liquid fatty acid ester comprising at least one fatty acid unit and at least one ethylene glycol unit and said second component may comprise a solid polyethylene glycol; or

said first component may comprise a liquid polyethylene glycol and said second compound is a solid fatty compounds selected from the group consisting of solid fatty acids and solid fatty soaps and solid fatty alcohols;

wherein when said solid nonionic surfactant is an ethoxylated fatty alcohol, then the HLB value is at least 13.

Preferably, when said solid fatty compound is a solid fatty acid, then the total amount of liquids is higher than the total amount of solids.

Preferably, the total amount of the first liquid component , at 25°C, is from 3 to 90 wt.% or from 20 to 80 wt.% or from 30 to 70 wt.% based on the total lotion composition and the total amount of the solid second component is from 10 to 97 wt.% or from 20 to 80 wt.% or from 30 to 70 wt.% based on the total lotion composition; and the weight ratio of first to second component is from 1:32 to 9:1 or from 1:9 to 9:1 1 or from 2:8 to 8:2 or from 3:7 to 7:3.

Preferably the lotion composition is hydrophilic and essentially non-aqueous. Non aqueous means, that the lotion compositions either contain no water or they contain water only in minor amounts such as less than 5 wt.% or even less than 1 wt.%. However, these amounts refer to the lotion composition at the time when the absorbent article is produced, i.e. to the time the lotion composition is applied onto the absorbent article. The lotion compositions of the present invention may be rather hygroscopic, and thus may be able to take up a significant amount of water from the surrounding atmosphere, particularly in an environment with high relative humidity. Thus, when the absorbent article has been stored for a relatively long time, such as several months or even years, it is possible that the amount of water contained in the lotion composition has increased to be more than 5 wt%.

The lotion composition comprises a first component which is liquid at 25°C and a second component which is solid at 25°C, for reducing the adherence of feces or menses to the human skin; wherein the first component comprises one or more compounds selected from the group consisting of

> (a) liquid polyethylene glycol;
> (b) liquid, polyhydric alcoholic solvents;
> (c) liquid fatty acid esters comprising at least one fatty acid unit and at least one ethylene glycol unit; and
> the second component comprises one or more compounds selected from the group consisting of
> (d) solid polyethylene glycol;
> (e) solid nonionic surfactants with HLB value of at least 10;
> (f) solid fatty compounds selected from the group consisting of solid fatty acids, solid fatty soaps and solid fatty alcohols;
> (g) ethoxylated natural oils and fats or propoxylated natural oils and fats, such as PEG-150 jojoba.

In one embodiment the lotion composition comprises a first component which is liquid at 25°C and a second component which is solid at 25°C, for reducing the adherence of feces or menses to the human skin; wherein the first component comprises one or more compounds selected from the group consisting of methoxyisopropanol, propyl ether, dipropylene glycol butyl ether, methyl propanediol, propylene carbonate, watersoluble polypropylene glycols, glycerin, sorbitol, hydrogenated starch hydrolysate, silicone glycols, and liquid PEG derivatives; and the second component comprises one or more components from the group consisting of

> (a) solid polyethylene glycol;
> (b) solid nonionic surfactants with HLB value of at least 10;
> (c) solid fatty compounds selected from the group consisting of solid fatty acids, solid fatty soaps and solid fatty alcohols;
> (d) ethoxylated natural oils and fats or propoxylated natural oils and fats, such as PEG-150 jojoba.

In one embodiment the liquid component is a polyethylene glycol having a molecular weight (weight average) of 100 to less than 720, preferably from 350 to 700. It may be preferred, that the lotion composition comprises from 20% to 80% by weight, or 30% to 70% by weight, or 40% to 60% by weight of this liquid polyethylene glycol. For example 50% by weight of Polyglycol 400.

In one embodiment the solid component is a polyethylene glycol having a molecular weight (weight average) of above 720, e.g. from 720 to 100000, or from 950 to 30000. It may be preferred, that the lotion composition comprises from 20% to 80% by weight, or 30% to 70% by weight, or 40% to 60% by weight of this liquid polyethylene glycol. For example 50% by weight of Polyglycol 4000.

In one embodiment the solid component is a solid nonionic surfactant, preferably a solid polyethylene glycol fatty alcohol ethers having the general formula $R(OCH_2CH_2)_nOH$, where R represents an alkyl group or a blend of alkyl groups, with for example 8 to 30 or 12 to 22 carbon atoms, and n is the degree of ethoxylation, e.g. 2 to 200. It may be preferred, that the lotion composition comprises from 20% to 80% by weight, or 30% to 70% by weight, or 40% to 60% by weight of this liquid polyethylene glycol. For example 50% by weight of Steareth-100.

The lotion composition may in one embodiment additionally comprise particulate material described herein after. Typically the particulate material is comprised by the component being solid at 25°C.

Liquid polyhydric alcoholic solvents

Liquid polyhydric alcoholic solvents, when used herein, are organic compounds having at least 2 carbon atoms and at least two alcoholic hydroxy groups and which are liquid at 25°C, excluding for the purpose of the invention polyethylene glycols, polypropylene glycols and derivatives, as described herein below, as separate preferred group. Examples are glycerol, ethylene glycol, diethylene glycol, propylene glycol, butylene glycol, dipropylene glycol, methyl propanediol and derivatives thereof, including for example mono- or di- end capped diethylene glycols, mono- or di- end capped dipropylene glycols, mono- or di- end capped ethylene glycols and mono- or di- end capped propylene glycols, having end-capped units as described above.

Preferred liquid compounds herein include: ethoxylated fatty acids, such as PEG-8 laurate, available for example as Lipopeg 4-L from Lipo Chemicals; ethoxylated fatty ester (oil), such as a PEG-25 castor oil, for example available as hetoxide C-25 from Global-Seven Inc.; Glycerol esters, such as for example a PEG-10 polyglyceryl-2 laurate, available for example as Hostacerin DGL from Clariant Corp.; Lecithin, such as available as Alcolec BS from American Lecithin Co.; polymeric surfactants such as a C8-C10 alkyl polysaccharide ether, available for example as Glucopan 225 DK from Cognis Corp.); Sorbitan derivatives such as POE (20) sorbitan monopalmitate available for example from Croda Inc.;sucrose and glucose esters and derivatives, such as alkyl polyglucoside, available for example as Simulsol AS48 from Seppic Inc.

Liquid polyethylene glycols and derivatives and liquid polypropylene glycol and derivatives.

Liquid polyethylene glycols and derivatives are liquid at 25°C. The polyethylene glycols (PEG's) are made from at least 3 units of ethylene glycol and have the general formula HO-(CH2-O-CH2-0)$_x$-H with x being a number of from 3 to 15 or from 8 to 12. The molecular weight (weight average) is from 100 to less than 720, preferably from 100 or 350 to 700. Typical liquid polyethylene glycols are known as PEG-4, PEG-6, PEG-7, PEG-8, PEG-9, PEG-10, PEG-12 and PEG-14. Suitable trade products are for example Polyglykol 400 of Clariant with an average molecular weight of 380 to 410 or Polyglykol 600 with an average molecular weight of 570 to 630.

Liquid PEG and PPG derivatives may include esters and ethers of PEG and PPG. Liquid derivates of PEG and PPG include in particular PEG's and PPG's (for example as described above) having however one or more (mono or di end capped, respectively) end cap groups, derived from an organic compound capable of reacting with a hydroxyl group. Preferred end cap groups include methyl, ethyl, propyl, isopropyl, butyl, isobutyl, tert-butyl and benzyl, for example mono- or di-methyl or- ethyl end capped PEG or PPG. In some embodiment, methyl may be a preferred end-capping unit. Further polypropylenes and end capping units useful in this invention are described in co-pending application US60/901793, filed 16 February 2007.

A preferred liquid mono-end capped PEG is for example a polyethylene glycol monomethyl ether, such as available as Polyglykol M400 from Clariant Corporation. A preferred liquid PEG includes also a Polyethylene glycol dimethyl ether with a MW of 500 (available from Sigma Aldrich).

Also useful herein are liquid ethylene oxide - propylene oxide copolymers and polyethylene - polypropylene block copolymers (EO-PO block copolymers), such as Genapol PF10- a EO-PO block copolymer from Clariant Corp.)

Liquid alkylene (e.g. ethylene) glycol fatty acid esters

Suitable liquid alkylene or preferably ethylene glycol fatty acid esters are for example the esters of one or more alkylene glycol units, preferably ethylene glycol units, and one or two fatty acids.

Preferred compounds have the general formula

R$^1$-(OCH2CH2)$_m$O-R$^2$ where R$^1$ and R$^2$ are hydrogen or fatty acid residues with e.g. from 6 to 30 or from 8 to 22 carbon atoms and can be the same or different with the proviso that not both are hydrogen; and m is a number of at least 1. Preferably, R1 and R2 are different and m is 1, 2, or 3. Typical ethylene glycol esters are known for example as diethylene glycol diethylhexanoate/ diisononanoate, diethylene glycol diisononanoate, diethylene glycol dilaurate, diethylene glycol dioctanoate/diisononanoate and diethylene glycol distearate. Suitable trade product mixtures containing ethylene glycol esters are for example DERMOL MO or DERMOL 489. Preferred are wax esters which are liquid at room temperature (25°C). They may be derived from natural sources such as jojoba oil, comprising docosenyl eicosenoate, eicosenyl eicosenoate and eicosenyl docosenoate.

Solid polyethylene glycol and polypropylene glycols and derivatives thereof

Solid polyethylene glycols, polypropylene glycols and derivatives thereof are solid (or semi-solid- as defined above) at 25°C, as defined herein. The solid polyethylene glycols are typically made from at least 16 units of ethylene glycol and have the general formula HO-(CH2-O-CH2-O)$_y$-H with y being a number of at least 16, e.g. from 20 to 220 or from 40 to 150. The molecular weight (weight average) is above 720, e.g. from 720 to 100000, or from 950 or 1500 or 2000 or 2700 to 30000. Typical solid polyethylene glycols are known as PEG-20, PEG-32, PEG-40, PEG-45, PEG-55, PEG-60, PEG-75, PEG-90 and PEG-100. Suitable trade products are for example Polyglykol 3000 of Clariant with an average molecular weight of 2700 to 3000 or Polyglykol 4000 with an average molecular weight of 3700 to 4500.

Solid PEG and PPG derivatives may include esters and ether derivates of PEG's and PPG's. Solid derivatives include in particular PEG's and PPG's (for example as described above) having one or more end cap groups (mono or di end capped, respectively), such as those described above.

For example, a solid mono-end capped PEG such as Polyglykol M4000 (polyethylene glycol monomethyl, from Clariant Corporation) may be used and/ or a solid di-endcapped PEG such as Polyethylene glycol dimethyl ether MW2000

6

(from Sigma Aldrich) may be used.

Also useful herein may be solid EO-PO copolymers and EO-PO block copolymers, such as for example Genapol PF80, an EO-PO block copolymer from Clariant Corp.

Solid nonionic surfactants

Suitable solid nonionic surfactants with an HLB value of at least 10 include solid PEG derived nonionic surfactants, solid polyalkylene glycol fatty alcohol ethers, such as solid polyethylene glycol fatty alcohol ethers or for example solid polyethoxylated fatty alcohols. The fatty alcohols unit may have from 8 to 30 carbon atoms, preferably from 12 to 22 carbon atoms. The average degree of alkoxylation, e.g. ethoxylation, may be from 2 to 200, preferably at least 10, at least 20 or at least 30. Preferably, these surfactants are nonionic surfactants with HLB values of at least 10, or at least 12 or at least 13, up to for example 17. Polyethylene glycol fatty alcohol ethers have the general formula $R(OCH2CH2)_nOH$, where R represents an alkyl group or a blend of alkyl groups with for example 8 to 30 or 12 to 22 carbon atoms and n is the degree of ethoxylation, e.g. 2 to 200. Suitable PEG derived surfactants include PEG-12 stearate, PEG-100 stearate, for example available as Tego Acid S 100 P from Evonik/ Degussa.
Suitable trade products include also for example BRIJ 76, BRIJ 78 and BRIJ 700 (Steareth 100, available from Croda Inc.). Other preferred surfactants include Ceteraeth-10, Ceteareth-20, Polysorbate- 65. Also used may be Laureth 23.

Suitable fatty alcohol fatty acid esters are esters of a C10- to C30 fatty alcohol with a C10- to C30-fatty acid. They have the general formula $R^3-CO-O-R^4$ where $R^3-CO$ is a C10- to C30 fatty acid residue and $O-R^4$ is a C 10- to C30 fatty alcohol residue. They may be saturated or unsaturated.

Other suitable nonionic surfactant are e.g. ethoxylated alcohols, ethoxylated fatty acids, ethoxylated fatty esters and oils, glycerol esters; sucrose and glucose esters and their derivatives, glucosides, sorbitan derivatives, such as sorbitan monoplamitate.

Other preferred compounds include PEG oils, like PEG40 hydrogenated caster oil, PEG-20 sorbitan monooleate, PEG-200 castor oil, available for example as Hetoxide C-200 from Global-Seven Inc.; glycerol esters such as a decaglycerol mono/dioleate, available for example as Caprol PGE860 from Abitec Corp.; lecithin derivatives, such as soy phosphatides, such as available as Alcolec Powder from American Lecithin Co.; sorbitan derivatives, such as Polysorbate 65, such as available as Liposorb TS-20 from Lipo Chemicals; sucrose and glucose esters and derivatives such as succinoglycan, available for example as Rheozan from Rhodia, Inc.

Solid fatty compounds:

The solid fatty compounds are selected from the group consisting of fatty acids, solid fatty soaps and solid fatty alcohols. The solid fatty compounds are solid at (or at least semi-solid according to the method described herein, at 25°C). The fatty compounds preferably have from 10 to 30 or from 12 to 22 carbon atoms. The fatty compounds can be saturated or unsaturated and they can be linear or branched. Preferred are saturated, linear fatty compounds. Examples of solid fatty acids are decanoic acid, lauric acid, myristic acid, palmitic acid, stearic acid, arachidic acid or behenic acid. Preferred solid fatty alcohols are linear, unsaturated 1-alkanols with at least 12 carbon atoms. Examples of solid fatty alcohols are lauryl alcohol, myristyl alcohol, cetyl alcohol, stearyl alcohol, arachidyl alcohol or behenyl alcohol. The solid fatty soaps are metallic soaps which are metal salts of fatty acids. The fatty acid components of the fatty soaps are the same as mentioned above. Suitable metal cations are sodium, potassium, lithium, aluminium, magnesium, calcium, mangan, iron, zirconium, cerium, zink, cobalt or vanadium. Preferred are metallic soaps with low water solubility such as the calcium or magnesium salts, e.g. calcium stearate.

Exemplary compositions:

It may be that the first liquid component comprises one or more compounds selected from the group consisting of: methoxyisopropanol, propyl ether, dipropylene glycol butyl ether, methyl propanediol, propylene carbonate, ethylene glycols, diethylene glycols, propylene glycols, dipropylene glycols, glycerin, sorbitol, hydrogenated starch hydrolysate, silicone glycols, or any of the above mentioned or exemplified polyethylne glycol or derivatives, polypropylene glycol or derivatives, polyethylene glycol derived surfactants, polypropylene derived surfactants, ethylene glycol or derivatives, propylene glycol or derivatives, diethylene glycol or derivatives and dipropylene glycol or derivatives, as described herein. The second, solid component may comprise for example one or more solid compounds (as defined above) of the group including: solid polyethylene glycol or derivatives thereof; solid polypropylene glycol or derivatives thereof; solid nonionic surfactants with HLB value of at least 10; solid fatty compounds selected from the group consisting of solid fatty acids, solid fatty soaps and solid fatty alcohols; solid PEG derived surfactants; solid PPG derived surfactants; ethoxylated natural fats or propoxylated natural fats, such as PEG-150 jojoba.

Exemplary lotion compositions may be such that: said first liquid component may comprise a liquid polyethylene glycol and said second component may comprise a solid nonionic surfactant with an HLB value of at least 10, provided that when said solid nonionic surfactant is an alkoxylated (e.g. ethoxylated) fatty alcohol, then the HLB value is at least 13; or said first component may comprise a liquid fatty acid ester comprising at least one fatty acid unit and at least one ethylene glycol unit and said second component may comprise a solid polyethylene glycol; or

said first component may comprise a liquid polyethylene glycol and said second compound is a solid fatty compounds selected from the group consisting of solid fatty acids and solid fatty soaps and solid fatty alcohols.

Preferably, when said solid fatty compound comprises a solid fatty acid, then the total amount of liquids is higher than the total amount of solids.

In one embodiment the liquid component is a polyethylene glycol having a molecular weight (weight average) of 100 to less than 720, preferably from 350 to 700. It may be preferred, that the lotion composition comprises from 20% to 80% by weight, or 30% to 70% by weight, or 40% to 60% by weight of this liquid polyethylene glycol. For example 50% by weight of polyethylene glycol with a MW of 400, also referred to as Polyglycol 400.

In one embodiment the solid component is a polyethylene glycol or derivative, where appropriate, having a molecular weight (weight average) of above 720, e.g. from 720 to 100000, or from 950 to 30000, or from 3000 to 20000 or to 10000. It may be preferred, that the lotion composition comprises from 20% to 80% by weight, or 30% to 70% by weight, or 40% to 60% by weight of this liquid polyethylene glycol, for example 50% by weight of Polyglycol 4000.

In one embodiment the solid component is a solid nonionic surfactant, preferably a solid polyethylene glycol fatty alcohol ethers having the general formula $R(OCH_2CH_2)_nOH$, where R represents an alkyl group or a blend of alkyl groups, with for example 8 to 30 or 12 to 22 carbon atoms, and n is the degree of ethoxylation, e.g. 2 to 200. It may be preferred, that the lotion composition comprises from 20% to 80% by weight, or 30% to 70% by weight, or 40% to 60% by weight of this liquid polyethylene glycol, for example 50% by weight of Steareth-100.

Particulate material

In one embodiment, the lotion composition additionally comprises at least one particulate material for further reducing the adherence of feces or menses to the skin. The particulate material is particulate during application onto the absorbent article. The particulate material is also such that it remains particulate when in contact with the skin and/ or when in contact with urine, menses or feces. Hence, the particulate material is water-insoluble and it has a melting temperature above the processing temperature of the lotion composition, as described above.

The particulate material may have any mean particle size between 1 nanometer to 2 mm, preferably between 1 nanometer to 500 micrometers, more preferably between 0.1 micrometer to 2mm, and still more preferably between 50 nanometers to 1 micrometer, or any range or individual value within any of the ranges set forth herein. Preferably, the minimum mean particle size is at least 0.1 micrometer or preferably at least 1 micrometer, or preferably at least 10 micrometers, or more preferably at least 20 micrometers, and preferably up to about 500 micrometers or in some embodiments up to about 100 micrometers, and further in other embodiments up to about 30 micrometers. In one embodiment, it may be preferred that the lotion composition to be applied and/or the applied coating comprises particles whereof less than 25% of the particles have an equivalent diameter of greater than 100 microns. In another embodiment, it may be preferred that the lotion composition to be applied and/or the applied coating comprises particles whereof less than 25% of the particles have an equivalent diameter of less than 5 microns. In yet another embodiment, it may be preferred that the lotion composition to be applied and/or the applied coating comprises particles whereof less than 25% of the particles have an equivalent diameter of less than 100 microns.

The particle material may be present in the lotion composition at a level from 0.05% to 25% (by weight of the lotion composition), preferably from 0.05% to 15%, more preferably from 0.05% to 5%, or from 0.1% to 25%, or more preferably from 0.25% to 20%, but typically from 0.5% to 10% or even up to 5% by weight.

Suitably, the particles may have a density between about 0.5 gram/cm$^3$ and about 2.5 gram/cm$^3$. Preferably, the density is between about 0.5 gram/cm$^3$ and about 2.0 gram/cm$^3$, and more preferably between 0.8 gram/cm$^3$ and about 1.5 gram/cm$^3$. In one embodiment, the density may preferably be less than about 1 gram/cm$^3$ so as to minimize particle settling and the density is greater than about 0.8 gram/cm$^3$ so as to minimize particle floatation.

In one embodiment, the lotion composition may comprise inorganic particles, including alumina silicates, silicates, silicas, mica and/ or talc. Clays may also be used. However, in the present invention it may be preferred that the particulate material is an organic material. Preferably, the particles are a non-active and/ or non-reactive material. The particles may be porous, or non-porous. The particles may have any shape, but preferably they have a smooth surface, and they may be preferably spherical or plate-like particles. The particles may comprise a coating agent on their surface or part thereof, for example a surfactant to change its properties, e.g. hydrophilicity. The particles, in particular when they are oleofinic, may include a melt-additive, which is added during the manufacturing of the particles.

Suitable materials include but are not limited to: polystyrene particles, polypropylene and/ or polyethylene (co) polymer particles, polytetrafluoroethylene particles, polymethylsilsesquioxane particles, nylon particles. Suitable commercially available particulate materials include but are not limited to: polyethylene particles, available from Honeywell International of Morristown, NJ under the trade name ACUMIST; polymethyl methacrylate particles (microspheres), available from KOBO of South Plainfield, NJ as BPA; lactone cross polymer particles (microspheres), available from KOBO as BPD; nylon 12 particles (microspheres), available from KOBO as NYLON SP; polymethylsilsesquioxane particles (microspheres), available from KOBO as TOSPEARL; cellulose particles (microspheres), available from KOBO as CELLO-BEADS; polytetrafluoroethylene powders, available from Micro Powders, Inc. of Tarrytown, NY as MICROSLIP; blends of natural wax and micronized polymers as are available form Micro Powders as MICROCARE and particles of a copolymer of vinylidene chloride, acrylonitrile and methylmethacrylate available as EXPANCEL from Expancel, Inc. of Duluth, GA.. Micronized waxes, such as are available from Micro Powders as MICROEASE may also be incorporated. Preferred are polyolefin particles (powders) as are available from Equistar Chemical Corp. Houston, TX as MICROTHENE. Particularly preferred is MICROTHENE FN510-00 from Equistar.

## Cuffs of the present invention

The invention provides an absorbent article with one or more cuffs having a hydrostatic head of at least 30 mbar and/ or a strike through time of at least 600 seconds; or said one or more cuffs comprise a material having a hydrostatic head of at least 30 mbar and/ or a strike through time of at least 600 seconds. The cuff referred to herein may be an anal cuff, a barrier cuff or a leg cuff. The article may comprise more than one cuff, or a combination of such cuffs. In one embodiment, the article comprises a pair of barrier cuffs and/ or a pair of leg cuffs, having (or comprising a material having) the hydrostatic head values and/ or strike through values as described herein.

As used herein "barrier cuffs" refer to cuffs designed to form a barrier to fluids; e.g. stopping or decreasing the amount of fluids passing through such a cuff. Typcially, the article comprises a pair of barrier cuffs, each cuff of said pair begin positioned along a longitudinal edge of the absorbent core.

Leg cuffs, as used herein, are typically comprised along the longitudinal side edges of the article, i.e. the article thus having a at least one leg cuff along each longitudinal side edge, and being in use in contact with the legs of the user.

Suitable cuffs in general are described in for example U.S. 3,860,003; U.S 4,808,178 and 4,909; U.S. 4,695,278 and 4,795,454. The cuffs may also be made of nonwoven materials and they are preferably hydrophobic.

In one embodiment the absorbent article according to the present invention comprises one pair of barrier cuffs.

The absorbent article may additionally comprise leg cuffs.

The cuff or cuffs of the article of the invention may comprise a nonwoven material (also referred to as cuff material) as described herein, for example such that at least 40%, or at least 60, or at least 80, or 100% of the surface area of the cuff comprises said nonwoven material. This nonwoven material may comprise one or more nonwoven webs. Each web may comprise one or more nonwoven layers for example at least 2, or at least 3 or at least 4 or at least 5 nonwoven layers. In one embodiment each web consists of 5 nonwoven layers. In one embodiment the cuffs comprise one nonwoven web.

The layers may be spunbond nonwoven layers (S), meltblown nonwoven layers (M) or nano-fiber nonwoven layers (N). In one embodiment at least the skinfacing surface and/ or at least the outer surface of the cuff material comprises a spunbond web. The presence of at least one or more meltblown or nano-fiber layers in the web may be preferred. Without being bound by theory it is believed that meltblown layers cause an enhancement of the barrier properties of the cuff material due to the good coverage per basis weight and hydrophobicity. These properties may be even more pronounced for the nano-fibers due to reduced fiber size as compared to meltblown fibers.

In another embodiment a web comprises at least 3, or at least 4, or at least 5 nonwoven layers whereof at least two are spunbond nonwoven layers.

In one embodiment in a web comprising at least 3 nonwoven layers the outermost layers are spunbond, any remaining nonwoven layers may be either meltblown or nano-fiber nonwoven layers or a combination thereof. Examples for such sequences are SMS, SMMS, SMMMS, SNS or SMNMS.

The basis weights given herein are values for weight per area of cuff material, nonwoven webs or nonwoven layers respectively. If the cuff material comprises any coating, the basis weights given herein include the weight of the coating (e.g. hydrophobic surface coatings as described herein).

The basis weight of a nonwoven meltblown layer may be at least 1 $g/m^2$ or at least 1.5 $g/m^2$ or at least 2 $g/m^2$, for example 2.5 $g/m^2$; in one embodiment the sum of the basis weights of all nonwoven meltblown layers comprised by the cuff material equals up to at least 5 $g/m^2$ or at least 7 $g/m^2$, for example 7.5 $g/m^2$. It may be preferred, that the basis weight of a nonwoven meltblown layer is less then 20 $g/m^2$ or 15 $g/m^2$.

The basis weight of a nonwoven nano-fiber layer may be at least 1 $g/m^2$ or at least 1.5 $g/m^2$ or at least 2 $g/m^2$, for example 2.5 $g/m^2$. It may be preferred, that the basis weight of a nonwoven nano-fiber layer is less then 20 $g/m^2$ or 15 $g/m^2$.

The basis weight of a nonwoven spunbond layer may be at least 3 $g/m^2$ or at least 5 $g/m^2$ or at least 7 $g/m^2$, for example 7.3 $g/m^2$ in one embodiment the sum of the basis weights of all nonwoven spunbond layers comprised by the cuff material equals up to at least 10 $g/m^2$ or at least 12 $g/m^2$ or at least 14 $g/m^2$, for example 14.6 $g/m^2$. It may be

preferred, that the basis weight of a nonwoven spunbond layer is less then 40 g/m$^2$ or 30 g/m$^2$.

In one embodiment the basis weight of a nonwoven web comprising one or more nonwoven layers may be at least 19 g/m$^2$ or at least 21 g/m$^2$ or at least 22 g/m$^2$, for example 22.1 g/m$^2$. It may be preferred, that the basis weight of said nonwoven web is less then 80 g/m$^2$ or 60 g/m$^2$.

In one embodiment, the nonwoven web comprises nano-fibers that may have an average diameter of 1.0 microns or less, or 0.8 microns or less, or 0.6 microns or less. The nonwoven web may comprise at least one nonwoven layer of which at least one or more or each nonwoven layer comprises such nano-fibers. The nonwoven web thereof may for example comprise at least 2 g/m$^2$ of nano-fibers, or at least 3 g/m$^2$ or at least 5 g/m$^2$ of nano-fibers. The nano-fibers may be made by known melt fibrillation methods or melt film fibrillation methods, such as described in US6,315,806 and US6,695,992. Preferred nano-fiber webs and layers are described in co-pending application WO2005/103355.

The cuff or cuff material has typically a hydrostatic head value, determined as described herein, of at least at least 30 mbar, or at least 35 mbar, or at least 38 mbar, or optionally at least 40 mbar.

The cuff or cuff material may additionally or alternatively have a low surface tension strike through value, as determined by the method described herein, of at least 600 seconds, or at least 800 seconds, or even at least 1000 seconds.

A material is considered to have the above hydrostatic head and/ or low surface tension strike through values if it has these values at any part of the material, excluding areas comprising elastic material or edges attached to another material: i.e. the measurement is done on a sample that does not comprise elastic material or any another material. In embodiments comprising lotion (e.g. the hydrophilic lotion of the present invention) the areas comprising that lotion are excluded as well. In one embodiment the one or more cuffs comprising the cuff material described herein, have a surface that is free of elastic material or any other material of at least 2.5 cm x 2.5 cm.

The cuff or cuff material may further comprise a hydrophobic agent, such as a wax.

The cuff material or one or more webs comprised by the cuff material may comprise a barrier agent, also referred to as hydrophobic surface coating, as described below .

As mentioned above, preferred cuff herein are made of hydrophobic material and/ or are treated to be hydrophobic, with for example a hydrophobic surface coating (HSC). Preferred hydrophobic surface coatings are for example described in co-pending application US60/543,785, filed February 11, 2004. Preferably, the hydrophobic surface coating or masking facilitating agent comprises one or more silicone polymers and / or fluorinated polymers. Suitable silicone polymers are for example selected from the group consisting of silicone MQ resins, polydimethysiloxanes, crosslinked silicones, silicone liquid elastomers, and combinations thereof. Typically, the weight average of the molecular weight (MW) of such silicone polymers should be at least about 4000 MW, preferably at least about 10,000 MW, more preferably at least about 15,000 MW, even more preferably at least about 20,000 MW, and most preferably at least about 25,000 MW. Preferred polydimethylsiloxanes are selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof. Suitable fluorinated polymers are selected from the group consisting of telomers and polymers containing tetrafluoroethylene and/or perfluorinated alkyl chains. For instance, fluorinated surfactants, which are commercially available from Dupont under the tradename Zonyl®, are suitable for use herein. In particular, Zonyl® 321, 329, 8740, 9027, and 9360 are well suited for use in the present invention. Additionally, other Zonyl® materials include fluroadditives like micro-powders may be useful herein. These include, but are not limited to Zonyl® MP1100, MP1200, MP1400, MP1500J, MP1600N, TE-3667N (which is a water dispersion). Preferably, the coating is free of aminosilicones.

These materials are preferably deposited onto the cuff or cuffs in amounts of from at least about 0.01 gsm (gram of material/square meter of topsheet), more preferably from at least about 0.05 gsm, and most preferably from at least about 0.1 gsm.

## Absorbent articles

This invention refers to any absorbent articles such as such as diapers, adult incontinence articles, sanitary napkins In the following, a diaper is described as one embodiment of an absorbent article. However, as the skilled person is aware of, most of the components and materials described herein below are also applicable to other absorbent articles such as pant-like diapers.

A preferred absorbent article herein is a diaper that has a longitudinal axis and a transverse axis. The diaper has further an inner, body facing surface and an outer, garment facing surface opposed to the inner surface.

One end portion of the diaper is configured as a front waist region of the diaper. The opposite end portion is configured as a back waist region of the diaper. An intermediate portion of the diaper is configured as a crotch region, which extends longitudinally between the front and back waist regions. The crotch region is that portion of the diaper which, when the diaper is worn, is generally positioned between the wearer's legs.

The chassis of the diaper comprises the main body of the diaper. The chassis comprises a liquid pervious topsheet and a backsheet. The chassis further includes an absorbent core encased between the topsheet and the backsheet. The chassis has a periphery which is defined by the transverse outer edges of the chassis with longitudinal edges and end edges.

Typically, the absorbent article comprises one or more cuffs that extend in longitudinal direction along the longitudinal side edges of the absorbent article, or part thereof. One or more of these cuffs may be a cuff or cuffs according to the present invention as described herein. The cuff or cuffs are typically attached to said absorbent article with one longitudinal edge of said cuff, thus having a free longitudinal edge that can be positioned out of the X-Y plane (longitudinal/ transverse directions) of the article, i.e. in z-direction. The cuffs are typically mirror images of one another in the Y-axis of the article.

In one embodiment a part of these one or more cuffs may comprise the hydrophilic lotion composition described herein, wherein said part comprises less then 80% of the cuff material, or less then 60%, or less then 40%, or even less then 20%.

The backsheet may typically be a liquid impervious backsheet, as known in the art. In one embodiment, the liquid impervious backsheet comprises a thin plastic film such as a thermoplastic film having a thickness of about 0.01 mm to about 0.05 mm. Suitable backsheet materials comprise typically breathable material, which permit vapors to escape from the absorbent article while still preventing exudates from passing through the backsheet. Suitable backsheet films include those manufactured by Tredegar Industries Inc. of Terre Haute, IN and sold under the trade names X15306, X10962 and X10964. The backsheet, or any portion thereof, may be elastically extendable in one or more directions.

The topsheet is preferably compliant, soft feeling, and non-irritating to the wearer's skin. Further, the topsheet is liquid pervious permitting liquids (e.g., urine) to readily penetrate through its thickness. A suitable topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be made of natural fibers (e.g., wood or cotton fibers), synthetic fibers (e.g., polymeric fibers such as polyester, polypropylene, or polyethylene fibers) or from a combination of natural and synthetic fibers. The topsheet is preferably made of a hydrophobic material to isolate the wearer's skin from liquids which have passed through the topsheet and are contained in the absorbent core (i.e., to prevent rewet). If the topsheet is made of a hydrophobic material, preferably at least the body facing surface of the topsheet or a part thereof is treated to be hydrophilic so that liquids will transfer through the topsheet more rapidly. This diminishes the likelihood that body exudates will flow off the topsheet rather than being drawn through the topsheet and being absorbed by the absorbent core. The topsheet can be rendered hydrophilic by treating it with a surfactant. In a preferred embodiment, the topsheet is a nonwoven web that can provide reduced tendency for surface wetness and consequently facilitate maintaining urine absorbed by the core away from the user's skin, after wetting.

The backsheet may be directly or indirectly attached to or joined with the topsheet herein and/ or the barrier and/ or leg cuffs herein.

The absorbent core generally is disposed between the topsheet and the backsheet. The absorbent core may comprise any absorbent material that is generally compressible, conformable, non-irritating to the wearer's skin, and capable of absorbing and retaining liquids such as urine and other certain body exudates.

The absorbent core may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, cross-linked cellulosic fibers, tissue including tissue wraps and tissue laminates, absorbent foams, absorbent sponges, superabsorbent polymers, absorbent gelling materials, or any equivalent material or combinations of materials. The configuration and construction of the absorbent core may also be varied, e.g. the absorbent core may have varying caliper zones, a hydrophilic gradient, a superabsorbent gradient, or lower average density and lower average basis weight acquisition zones; or may comprise one or more layers or structures. The total absorbent capacity of the absorbent core should, however, be compatible with the design loading and the intended use of the diaper. Further, the size and absorbent capacity of the absorbent core may be varied to accommodate wearers ranging from infants through adults.

The topsheet of the absorbent article of the present invention can also be apertured, i.e. the topsheet has a plurality of apertures having an aperture size of at least about 0.2 mm$^2$. The topsheet has preferably an open area of at least about 10%, the open area being the sum of all apertures.

In one embodiment the article may comprise an anal cuff or topsheet, that has one or more openings that are large enough to let feces (or menses) pass to a void space underneath said topsheet or cuff. The anal or vaginal cuff may be a cuff according to the present invention as described herein.

For example, U.S. Patent Application No. 2006/0058766 A filed on September 13, 2005 discloses an absorbent article wherein the topsheet is provided with at least one opening adapted to receive fecal material, the topsheet and the opening thereof each having a front region and a back region. A void space between the absorbent core and the topsheet is provided and the absorbent article further comprises a genital coversheet, which in use covers the genitals, and which is positioned in, under or above said front region of the opening. Further suitable absorbent articles are disclosed e.g. in U.S. Patent 6,482,191; U.S. Patent Application No. 2004/0092902 A; U.S. Patent Application No. 2004/0092900 A; U.S. Patent Application No. 2004/0162538 A; and U.S. Patent Application No. 2006/0058765 A.

Further, the absorbent articles may comprise a front and back waist band and/ or a fastening system, typically joined to the waistband, as known in the art. Preferred fastening systems comprise fastening tabs and landing zones, wherein the fastening tabs are attached or joined to the back region of the diaper and the landing zones are part of the front region of the diaper.

**Test methods**

Water solubility

Water solubility of the lotion composition or any compound used to formulate the lotion composition is determined as follows: 100 mg starting amount (SA) of the lotion composition is applied to a glass slide (2.5 cm x 8 cm) of known weight, such that the lotion covers an area of 2.5 cm x 5 cm on the glass slide. The slide is then placed flat in a beaker containing 75ml of deionized water at 20°C. The water with the lotion composition therein is not stirred. After 4 hours the glass slide is removed from the beaker and put in an oven at 60°C, 0% RH (relative humidity) to remove the water. After drying it is weighted to determine the residual amount of lotion composition on the slide. The lotion composition of the present invention is water soluble if residual amount (RA) of lotion composition on the plate after drying is below 60%, more preferably below 20% and even more preferably below 10% (of the 100 mg that have been applied to the glass slide). These values correspond to a water solubility of at least 40%, more preferably at least 80% and even more preferably at least 90% which is determined as follows:

$$[(SA - RA)]/ \, SA \times 100\% = \text{water solubility (in \%)}$$

Such lotion compositions having relatively good water solubility are considered to be hydrophilic within the meaning of the present invention.

Hydrostatic head (hydrohead)

The hydrostatic head (also referred to as hydrohead) as used herein is measured with deionized water (DI water)

This test is performed as set out in co-pending application WO2005/112854A, conform the Inda/ Edana test WSP 80.6 (05). The water pressure is increased at a rate of 20mbar per minute in the testing using DI water (72mN/m).

A sample of 5 cm$^2$ is taken from the cuff or the cuff material. The sample should be free from elastic material or edges that are connected to other materials.

The test head used has a 2.5 cm diameter; the protective sleeve used has a 2.2 cm diameter.

Low surface tension Strike Through value method

The low surface tension strike through value referred to herein may be obtained by the Edana method WSP70.3 (05), except that a low surface tension liquid (see below) is used and a sample of 1 inch x 1 inch (25 mm x 25 mm) may be used. The sample should be free of elastic material or of edges that are connected to other materials.

The value obtained from this sample measurement is reported herein.

The low surface tension liquid is a liquid with a surface tension of 49 mN/m prepared as follows:

*49 mN/m (dynes/ cm) liquid preparation:*

A 10 litre canister with tap is cleaned thoroughly 3 times with 2 litres polyethylene and then 3 times with 2 litres distilled/deionized water.

Then, it is filled with 10 litres distilled/deionized water and stirred with a clean stirring bar for 2 h, after which the water is released via the tap.

A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.

The stirring bar is removed from the glass with a magnetic stick (without touching the solution) and then the Na cholate solution is poured into the 10 litres canister and more distilled/deionized water is added such that the concentration of the final solution is 3 g/l. This is further stirred with a stirring bar for 2 hours and then used.

This preparation of the solution carried out at 20°C.

The surface tension of the solution is measured and this should be 49 mN/m (+/- 2). (The surface tension may be determined by method: ASTM D1331-56 ("Standard test method for surface and interfacial tension of solution of surface active agents") using a Kruss K12 tensiometer.)

## Claims

**1.** An absorbent article to be worn by a wearer comprising a backsheet, a topsheet, an absorbent core, one or more cuffs and a lotion, wherein said lotion comprises a first component which is liquid at 25°C and a second component which is solid at 25°C; said first component comprising one or more compounds selected from the group consisting, of

(a) liquid polyethylene glycol or derivate;
(b) liquid polypropyle glycol or derivative
(c) liquid fatty acid esters comprising at least one fatty acid unit and at least one ethylene glycol unit;
and said second component comprising one or more compounds selected from the group consisting of
(d) solid polyethylene glycol, solid polypropylene glycol or end-capped or ether derivative of either,
(e) solid nonionic surfactants with HLB value of at least 10;
(f) solid fatty compounds selected from the group consisting of solid fatty acids, solid fatty soaps and solid fatty alcohols; and
(g) ethoxylated natural oils and fats or propoxylated natural oils and fats, such as PEG- 150 jojoba; and

wherein said one or more cuffs comprise one or more nonwoven webs, each comprising one or more nonwoven layers; wherein said at least one nonwoven web comprises least 4 layers, and wherein said lotion comprises at least 20 % by weight of said first component or at least 20 % by weight of said second component; and wherein said one or more cuffs, each comprise at least one nonwoven web, whereof at least one is a spunbond nonwoven layer and at least one is a meltblown or nano-fiber nonwoven layers.

**2.** The absorbent article according to one or more of the receding claims wherein said cuffs comprise a cuff material, and said cuff or said cuff material has a hydrostatic head of at least 35 mbar and/ or a strike through time of at least 1000 seconds.

**3.** The absorbent article according to one or more of the preceding claims wherein said lotion is applied on the topsheet.

**4.** The absorbent article according to one or more of the preceding claims, wherein said lotion is applied in an amount of at least 1 gsm and said cuffs, or nonwoven layers comprise a coating of at least one barrier agent selected from the group consisting of silicone polymers and fluorinated polymers or a combination thereof.

**5.** The absorbent article according to one or more of the preceding claims, wherein said nonwoven web has a basis weight of at least 19 g/m$^2$ and comprises at least 5 g/m$^2$ of spunbond fibers and further comprises at least 2 g/m$^2$ of meltblown fibers and/ or at least 2g/m$^2$ nano fibers.

**6.** The absorbent article according to one or more of the preceding claims, wherein the cuff comprises two spunbond (S) and three meltblown (M) nonwoven layers wherein the sequence of these layers is SMMMS.

**7.** The absorbent article according to one or more of the preceding claims wherein the absorbent article is an incontinence article, a diaper or a pant like diaper.

## Patentansprüche

**1.** Absorptionsartikel, der von einem Träger zu tragen ist, umfassend eine Unterschicht, eine Oberschicht, einen Absorptionskern, ein oder mehrere Bündchen und eine Lotion, wobei die Lotion einen ersten Bestandteil umfasst, der bei 25 °C flüssig ist, und einen zweiten Bestandteil, der bei 25 °C fest ist; wobei der erste Bestandteil eine oder mehrere Verbindungen umfasst, die ausgewählt sind aus der Gruppe bestehend aus

(a) flüssigem Polyethylenglycol oder einem Derivat;

(b) flüssigem Polypropylenglycol oder einem Derivat

(c) flüssigen Fettsäureestern, umfassend mindestens eine Fettsäureeinheit und mindestens eine Ethylenglycoleinheit;

und wobei der zweite Bestandteil eine oder mehrere Verbindungen umfasst, die ausgewählt sind aus der Gruppe bestehend aus

(d) festem Polyethylenglycol, festem Polypropylenglycol oder einem endverkappten Derivat oder Etherderivat von einer dieser Verbindungen;

(e) festen nichtionischen Tensiden mit einem HLB-Wert von mindestens 10;

(f) festen Fettverbindungen, ausgewählt aus der Gruppe bestehend aus festen Fettsäuren, festen Fettseifen und festen Fettalkoholen; und

(g) ethoxylierten natürlichen Ölen und Fetten oder propoxylierten natürlichen Ölen und Fetten wie PEG-150 Jojoba; und

wobei das eine oder die mehreren Bündchen eine oder mehrere Vliesbahnen umfassen, die jeweils eine oder mehrere Vliesschichten umfassen; wobei die mindestens eine Vliesbahn mindestens 4 Schichten umfasst; und wobei die Lotion zu mindestens 20 Gew.-% den ersten Bestandteil oder zu mindestens 20 Gew.-% den zweiten Bestandteil umfasst; und wobei das eine oder die mehreren Bündchen jeweils mindestens eine Vliesbahn umfassen, wovon mindestens eine eine Spinnvliesschicht ist und mindestens eine eine schmelzgeblasene Vliesschicht oder eine Nanofaser-Vliesschicht ist.

2. Absorptionsartikel nach einem oder mehreren der vorstehenden Ansprüche, wobei die Bündchen ein Bündchenmaterial umfassen und das Bündchen oder das Bündchenmaterial einen hydrostatischen Druck von mindestens 3,5 kPa (35 mbar) und/oder eine Durchgangszeit von mindestens 1000 Sekunden aufweist.

3. Absorptionsartikel nach einem oder mehreren der vorstehenden Ansprüche, wobei die Lotion auf die Oberschicht aufgetragen wird.

4. Absorptionsartikel nach einem oder mehreren der vorstehenden Ansprüche, wobei die Lotion in einer Menge von mindestens 1 Gramm pro Quadratmeter aufgetragen wird und die Bündchen oder Vliesschichten eine Beschichtung aus mindestens einem Sperrmittel umfassen, das ausgewählt ist aus der Gruppe bestehend aus Silikonpolymeren und fluorierten Polymeren oder einer Kombination davon.

5. Absorptionsartikel nach einem oder mehreren der vorstehenden Ansprüche, wobei die Vliesbahn ein Basisgewicht von mindestens 19 g/m$^2$ besitzt und mindestens 5 g/m$^2$ Spinnvliesfasern umfasst und ferner mindestens 2 g/m$^2$ schmelzgeblasene Fasern und/oder mindestens 2 g/m$^2$ Nanofasern umfasst.

6. Absorptionsartikel nach einem oder mehreren der vorstehenden Ansprüche, wobei das Bündchen zwei Spinnvliesschichten (S) und drei schmelzgeblasene M) Vliesschichten umfasst, wobei die Abfolge dieser Schichten SMMMS ist.

7. Absorptionsartikel nach einem oder mehreren der vorstehenden Ansprüche, wobei der Absorptionsartikel ein Inkontinenzartikel, eine Windel oder eine hosenartige Windel ist.

**Revendications**

1. Article absorbant destiné à être porté par un utilisateur comprenant une feuille de fond, une feuille de dessus, un corps absorbant, un ou plusieurs revers et une lotion, dans lequel ladite lotion comprend un premier composant qui est liquide à 25 °C et un second composant qui est solide à 25 °C ; ledit premier composant comprenant un ou plusieurs composés choisis dans le groupe constitué de

(a) polyéthylène glycol liquide ou dérivé ;

(b) polypropyl glycol liquide ou dérivé ;

(c) esters d'acide gras liquides comprenant au moins un motif acide gras et au moins un motif éthylène glycol et ledit deuxième composant comprenant un ou plusieurs composés choisis dans le groupe constitué de

(d) polyéthylène glycol solide, polypropylène glycol solide ou dérivé à extrémité coiffée ou éther de l'un ou l'autre ;

(e) agents tensioactifs non ioniques solides avec un rapport hydro-lipophile d'au moins 10 ;

(f) composés gras solides choisis dans le groupe constitué d'acides gras solides, savons gras solides et alcools

**14**

gras solides ; et

(g) huiles et graisses naturelles éthoxylées ou huiles et graisses naturelles propoxylées, telles que PEG-150 jojoba ; et

dans lequel lesdits un ou plusieurs revers comprennent une ou plusieurs nappes non tissées, comprenant chacune une ou plusieurs couches de non-tissé ; dans lequel ladite au moins une nappe non tissée comprend au moins 4 couches ; et dans lequel ladite lotion comprend au moins 20 % en poids dudit premier composant ou au moins 20 % en poids dudit deuxième composant ; et dans lequel lesdits un ou plusieurs revers comprennent chacun au moins une nappe non tissée, parmi lesquelles au moins une est une couche de non-tissé filée-liée et au moins une est une couche de non-tissé soufflée en fusion ou à nano-fibres.

2. Article absorbant selon l'une ou plusieurs des revendications précédentes, dans lequel lesdits revers comprennent un matériau de revers, et ledit revers ou ledit matériau de revers a une charge hydrostatique d'au moins 3,5 kPa (35 mbar) et/ou un temps de transpercement d'au moins 1 000 secondes.

3. Article absorbant selon l'une ou plusieurs des revendications précédentes, dans lequel ladite lotion est appliquée sur la feuille de dessus.

4. Article absorbant selon l'une ou plusieurs des revendications précédentes, dans lequel ladite lotion est appliquée en une quantité d'au moins 1 g/m$^2$ et lesdits revers ou couches de non-tissé comprennent un revêtement d'au moins un agent de barrière choisi dans le groupe constitué de polymères de silicone et polymères fluorés et leurs combinaisons.

5. Article absorbant selon l'une ou plusieurs des revendications précédentes, dans lequel ladite nappe non tissée a une masse surfacique d'au moins 19 g/m$^2$ et comprend au moins 5 g/m$^2$ de fibres filées-liées et comprend en outre au moins 2 g/m$^2$ de fibres soufflées en fusion et/ou au moins 2 g/m$^2$ de nano-fibres.

6. Article absorbant selon l'une ou plusieurs des revendications précédentes, dans lequel le revers comprend deux couches de non-tissé filées-liées (S) et trois soufflées en fusion (M) dans lequel l'ordre de ces couches est SMMMS.

7. Article absorbant selon l'une ou plusieurs des revendications précédentes, où l'article absorbant est un article pour l'incontinence, une couche ou une couche-culotte.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5968025 A, Roe
- WO 9705908 A
- WO 9705909 A
- US 2006140924 A
- US 3489148 A, Duncan
- WO 05035013 A
- WO 0064500 A
- WO 0064501 A
- US 2002 A
- US 120241 A
- US 6756520 B
- US 3860003 A, Buell
- US 5151092 A, Buell
- US 5221274 A, Buell
- US 5554145 A, Roe
- US 5246433 A, Hasse
- US 4253461 A, Strickland
- US 4597760 A
- US 4597761 A, Buell
- US 4704115 A
- US 4909802 A, Ahr
- US 4964860 A, Gipson
- WO 9211830 A, Noel
- US 60901793 B
- US 4808178 A
- US 4909 A
- US 4695278 A
- US 4795454 A
- US 6315806 B
- US 6695992 B
- WO 2005103355 A
- US 60543785 B
- US 20060058766 A
- US 6482191 B
- US 20040092902 A
- US 20040092900 A
- US 20040162538 A
- US 20060058765 A
- WO 2005112854 A